# EUROPEAN PATENT SPECIFICATION

(11) **EP 2 001 537 B1**
(45) Date of publication and mention of the grant of the patent: **09.02.2011**
(21) Application number: 07781365.7
(22) Date of filing: 04.04.2007
(51) Int. Cl.: A61M 11/00, A61M 15/00

(54) **VARIABLE DOSE INHALATION DEVICE**
INHALATIONSVORRICHTUNG FÜR VARIABLE DOSIERUNG
DISPOSITIF D'INHALATION À DOSES VARIABLES

(30) Priority: 05.04.2006 US 789290 P; 04.04.2007 US 696683
(43) Date of publication of application: 17.12.2008
(60) Divisional application: 10195741.3
(73) Proprietor: MicroDose Therapeutx, Inc., Monmouth Junction, NJ 08852 (US)
(72) Inventor: FLEMING, Scott, Ewing, New Jersey 08628 (US); GUMASTE, Anand, V., West Windsor, New Jersey 08550 (US); KRIKSUNOV, Leo, B., Ithaca, New York 14850 (US); AKERMAN, Adan, Monmouth Junction, New Jersey 08852 (US)
(74) Representative: Epping - Hermann - Fischer
(86) International application number: PCT/US2007/066005
(87) International publication number: WO 2007/121097

(56) References cited:
- EP-A- 1 166 812
- WO-A-02/04055
- WO-A-99/64095
- US-A1- 2002 129 812
- US-A1- 2002 138 588
- US-A1- 2004 111 467

## Description

The present invention relates generally to the field of inhalation devices. The invention has particular utility in inhalation devices that utilize vibration to facilitate suspension of therapeutic agents or drugs, either in powder or liquid form into an inhaled gas stream (e.g., inhaled air), and will be described in connection with such utility, although other utilities are contemplated.

Certain diseases of the respiratory tract are known to respond to treatment by the direct application of therapeutic agents or drugs. As these agents or drugs are most readily available in dry powdered form, their application is most conveniently accomplished by inhaling the powdered material through the nose or mouth. This powdered form results in better utilization of the agent or drug in that the agent or drug is deposited exactly at the site desired and where its action may be required; hence, very minute doses of the agent or drug are often equally as efficacious as larger doses administered by other means, with a consequent marked reduction in the incidence of undesired side effects including risk or under or over dose and cost. Alternatively, the agent or drug in this form may be used for treatment of diseases other than those of the respiratory system. When the agent or drug is deposited on the very large surface areas of the lungs, it may be very rapidly absorbed into the blood stream; hence, this method of application may take the place of administration by injection, tablet, or other conventional means.

It is the opinion of the pharmaceutical industry that the bioavailability of most drugs is optimum when the drug particles delivered to the respiratory tract are between about 1 to 5 microns in size. However, delivery of drug particles in this size range presents several issues:
(1) Small size particles develop an electrostatic charge during manufacturing and storage. This causes the particles to agglomerate or aggregate, resulting in clusters of particles, which have an effective size greater than about 5 microns. The probability of these large clusters making it to the deep lungs then decreases. This in turn results in a lower percentage of the drug being available to the patient for absorption.
(2) The amount of active drug that needs to be delivered to the patient may be of the order of just a few (e.g. 10s) of micrograms. For example, albuterol, in the case of a drug used in asthma, this is usually 25 to 50 micrograms. Current manufacturing equipment can effectively deliver aliquots of drugs in milligram dose range with acceptable accuracy. Therefore, the standard practice is to mix the active drug with an excipient filler or bulking agent such as lactose. This additive also makes the drug "easy to flow." This filler is also called a carrier since the drug particles also stick to these particles through electrostatic or chemical bonds. These carrier particles are very much larger than the drug particles in size. The ability of the dry powder inhaler to separate drug from the carrier is an important performance parameter in the effectiveness of the design.
(3) Active drug particles with sizes greater than about 5 microns will be deposited either in the mouth or throat. This introduces another level of uncertainty since the bioavailability and absorption of the drug in these locations is different from the lungs. Dry powder inhalers need to minimize the drug deposited in these locations to reduce the uncertainty associated with the bioavailability of the drug.

Prior art dry powder inhalers (DPIs) usually have a means for introducing the drug (active drug plus carrier) into a high velocity air stream. The high velocity airstream is used as the primary mechanism for breaking up the cluster of micronized particles or separating the drug particles from the carrier. Several inhalation devices useful for dispensing this powder form of medicament are known in the prior art. For example, in U.S. Patent Nos. 3,507,277; 3,518,992; 3,635,219; 3,795,244 and 3,807,400, inhalation devices are disclosed having means for piercing of a capsule containing a powdered medicament, which upon inhalation is drawn out of the pierced capsule and into the user's mouth. Several of these patents disclose propeller means, which upon inhalation aid in dispensing the powder out of the capsule, so that it is not necessary to rely solely on the inhaled air to suction powder from the capsule. For example, in U.S. Patent No. 2,517,482, a device is disclosed having a powder containing capsule placed in a lower chamber before inhalation, where it is pierced by manual depression of a piercing pin by the user. After piercing, inhalation is begun and the capsule is drawn into an upper chamber of the device where it moves about in all directions to cause a dispensing of powder through the pierced holes and into the inhaled air stream. U.S. Patent No. 3,831,606 discloses an inhalation device having multiple piercing pins, propeller means, and a self-contained power source for operating the propeller means via external manual manipulation, so that upon inhalation the propeller means aids in dispensing the powder into the stream of inhaled air. See also U.S. Patent No. 5,458,135.

The above description of the prior art is taken largely from U.S. Pat. No. 3,948,264 to Wilke et al, who disclose a device for facilitating inhalation of a powdered medication that includes a body portion having primary and secondary air inlet channels and an outlet channel. The secondary inlet channel provides an enclosure for a capsule containing the powdered medication and the outlet channel is formed as a mouthpiece protruding from the body. A capsule piercing structure is provided, which upon rotation puts one or more holes in the capsule so that upon vibration of the capsule by an electromechanical vibrator, the powdered drug may be released from the capsule. The piercing means disclosed in Wilke et al includes three radially mounted, spring-biased piercing needles mounted in a trochoidal chamber. Upon hand rotation of the chamber, simultaneous inward radial motion of the needles pierces the capsule. Further rotation of the chamber allows the needles to be retracted by their spring mountings to their original positions to withdraw the needles from the capsule. The electromechanical vibrator includes, at its innermost end, a vibrating plunger rod which projects into the intersection of the inlet channel and the outlet channel. Connected to the plunger rod is a mechanical solenoid buzzer for energizing the rod to vibrate. The buzzer is powered by a high-energy electric cell and is activated by an external button switch. According to Wilke et al, upon inhalation through an outlet channel and concurrent pressing of a switch to activate the electromechanical vibrating means, air is sucked through one or more inlet channels and the air stream through a secondary inlet channel raises the capsule up against a vibrating plunger rod. The capsule is thus vibrated rapidly with powder being fluidized and dispensed from the pierced holes therein. This technique is commonly used in manufacturing for dispensing powder through a hopper where the hopper is vibrated to fluidize the powder and move it through the hopper outlet. The pierced holes in the capsule represent the hopper outlet. The air stream through the inlet channel aids in withdrawal of powder from the capsule and carries this powder through the outlet channel to the mouth of the user. Wilke et al. further discloses that the electromechanical vibrator means may be placed at a right angle to the inlet chamber and that the amplitude and frequency of vibration may be altered to regulate dispensing characteristics of the inhaler.

The vibrator in Wilke et al.'s disclosed inhaler is an electromechanical device consisting of a rod driven by a solenoid buzzer. According to Wilke et al, this electromechanical means may be a motor driving a cam. A disadvantage of the inhaler implementation as disclosed by Wilke is the relatively large mechanical movement required of the rod to effectively vibrate the capsule. The large movement of the rod, usually around 100s of microns, is necessary due to the elasticity of the capsule walls and inertia of the drug and capsule.

Solenoid buzzers typically have operating frequencies less than five kHz. This operating frequency tends to be noisy and therefore is not desirable when incorporated into a dry powder inhaler from a patient's perspective. A further disadvantage of the electromechanical actuators of Wilke is the requirement for a high-energy source, thus requiring a large battery source or frequent changes of the battery pack for portable units. Both these features are not desirable from a patient safety and "ease of use" standpoint.

The inhaler of Wilke et al is primarily intended to reduce the amount of powder left behind in the capsule relative to other inhalers cited in the patent disclosure. However, Wilke et al does not address the need to deaggregate the powder into particle sizes or groups less than 6 microns in size as is required for effective delivery of the medication to the lungs; rather Wilke et al, like prior art inhalers continues to rely on the air stream velocity to deaggregate the powder ejected into the air stream, into particle sizes suitable for delivery to the lungs.

Another prior art inhalation device is disclosed in Bums et al U.S. Patent No. 5,284,133. In this device, a liquid medication is atomized by an ultrasonic device such as a piezo element. A stream of air, usually at a high velocity, or a propellant then carries the atomized particles to the patient. However, the energy required to atomize the liquid medication in the nebulizer is prohibitively high, making this approach for the delivery of drugs to the lungs primarily only feasible as a desktop unit.

The prior art devices therefore have a number of disadvantages including:
- The performance of the prior art inhalers depends on the flow rate generated by the user. Lower flow rate may not result in the powder being totally deaggregated and hence adversely affects the dose delivered to the patient.
- Inconsistency in the bioavailability of the drugs from dose-to-dose because of lack of consistency in the deaggregation process.
- Large energy requirements for driving electromechanical based inhalers, which increases the size of the devices.

Another disadvantage of the prior art devices is the capability to deliver only a fixed dose of the drug to the patient, while patient's needs with respect to the dosing of the drug can vary depending on the current status of the medical condition of the patient. For example, a diabetic patient may need different amounts of insulin based on measurement of glucose concentration in the patient's blood.

In U.S. 5,694,920, issued December 9, 1997, an inhaler is disclosed that utilizes vibration to facilitate suspension of powder into a gas that overcomes the aforesaid and other disadvantages and drawbacks of the above prior art. More particularly, the inhaler of our aforesaid patent includes a piezoelectric vibrator for vibrating the powder. A controller is provided for controlling supply (i.e., amplitude and/or frequency) of actuating electricity to the vibrator so as to cause vibration of the powder that is adapted to optimally suspend at least a portion of the powder into the gas. As described in our aforesaid patent, the controller may include a user-actuable control for permitting the user to select the vibration frequencies and/or amplitudes for optimally suspending in the gas the type of powder currently being used in the inhaler. The user-actuable control is pre-calibrated with the controller to cause the controller to adjust the frequency and/or amplitude of actuating electricity supplied to the vibrator to be that necessary for vibrating the type of powder selected by the user-actuable control in such a way as to optimally suspend at least a portion of the powder into the gas. The user-actuable control may include selection gradations in terms of the average size of the powder particles to be suspended in the gas, and/or in terms of desired vibration frequencies and amplitudes. Vibration frequency would be adjusted to at least about 12 kHz, in order to optimally suspend such commonly used powdered medications in the gas. Of course, vibration frequency and amplitude may be adjusted to optimize suspension of the powdered medication being used.

An electrostatic field that is established across the air stream, whereby by controlling the strength of the electrostatic field primarily only particle sizes of interest are introduced into the air stream, while larger size particles are left behind in the container. This reduces the inconsistency associated with the bioavailability of the drug because of the large particles being deposited into the mouth or throat as is common with devices described in prior art.
WO 02/04055 A1 and U.S. 2002/0129812 A1 disclose nebulizing devices for the delivery of therepeutic agents which are fluid. The compartments of the fluid therepeutic agents are accessed repeatedly, namely each time when an amount of agent is to be inhaled. A risk of exposure to oxygen and/or moisture is thus inherent to this kind of inhalation devices.
WO 99/64095 A2 discloses an inhalation devices requiring electronic circuitry for electronically controlling dosing.

EP 1 166 812 A1 discloses a blister pack for use with inhalation devices.
U.S. 6,142,146, issued November 7, 2000, discloses an inhaler with piezoelectric elements are designed to vibrate at different amplitudes and frequencies, i.e. so that, for example, two different drugs advantageously may be dispersed simultaneously from the same inhaler, without compromising performance or either drug. This permits delivery of two drugs that, while active together, may not readily be stored together. For example, an asthma inhaler may be provided containing both a bronchodilator, such as albuterol, and a steroid which may require different piezo settings.

Similarly, US Patent No. 6,684,879 issued February 3, 2004 to Coffee et al. teaches an inhaler using two or more piezoelectric resonators arranged to resonate at different frequencies to aerosolize liquid droplets.

The present invention provides an improvement over the prior art inhalation devices such as our aforementioned U.S. Patent No. 6,142,146. This invention allows the user to easily administer varying doses of a therapeutic agent or drug. As used herein, the terms "medication", "therapeutic agent", "agent" and "drug" are used interchangeably. Prior art inhalers only allowed the user to administer a single or extremely limited number of doses at once. It is the object of the present invention to allow the user to administer varying doses of one or more therapeutic agnts or drugs in a single or controlled number of inhalations. It is a further object of the invention to limit the number of inhalations necessary to administer a desired quantity of a medication or combination of different medications and thus to improve compliance and efficacy.

This object is solved by the inhalation devices of claim 1.

For example, in case of delivery of powered insulin in an inhaler, currently available inhalers for delivering powdered insulin are all single dose devices. However, a person suffering from diabetes may need varying doses of insulin, multiple times during a day, based on a measurement of their blood sugar level at that time. This means that the user either must carry several inhalation devices each delivering different doses, or the patient must take several puffs in succession in order to achieve a desired dosage. The inhaler of the present invention provides an efficient and convenient way to provide varying doses of insulin in one inhalation step.

In one embodiment, the invention provides for an inhaler with two (or more) vibrator mechanisms or piezoelectric elements and addressable dose packs. Thus, the inhaler of the present invention individual blisters of rapidly acting insulin with different dosage may be inserted into the inhaler to provide the needed dosage. For example, if the user needed 8 units of insulin, a blister with 5 units and a blister with 3 units could be loaded in the inhaler and dispensed in one shot. Thus, the inhaler of the present invention provides for the simple and effective administration of varying quantities of a medication without the multiple inhalations required by prior art inhalers.

In another embodiment of the invention, the inhaler contains two or more vibrator mechanisms or piezoelectric elements each located in separate powder dispensing chambers. The inhaler structure permits the user to insert individual blisters of a drug, which may contain the same or different size doses of medication, into the inhaler for one shot delivery. In a second embodiment of the inhaler, the two or more vibrator mechanisms or piezoelectric elements are located in the same powder dispensing chamber.

In still yet another embodiment of the invention, two (or more) cartridge strips are inserted in the back of the inhaler. Each strip contains one or a plurality blisters containing a drug or medicine. The user selects desired dosage of the medicine or drug by accessing one or a plurality of blisters on one or both (or more) cartridge steps.

In yet another embodiment of the invention, individual blisters of a drug or medicine are inserted using a fixture or tool which permits selection and handling of blisters without finger contact.

In yet another embodiment of the invention, the blisters are packaged on a spool or rotatable cartridge and dropped or placed one at a time into the inhaler.

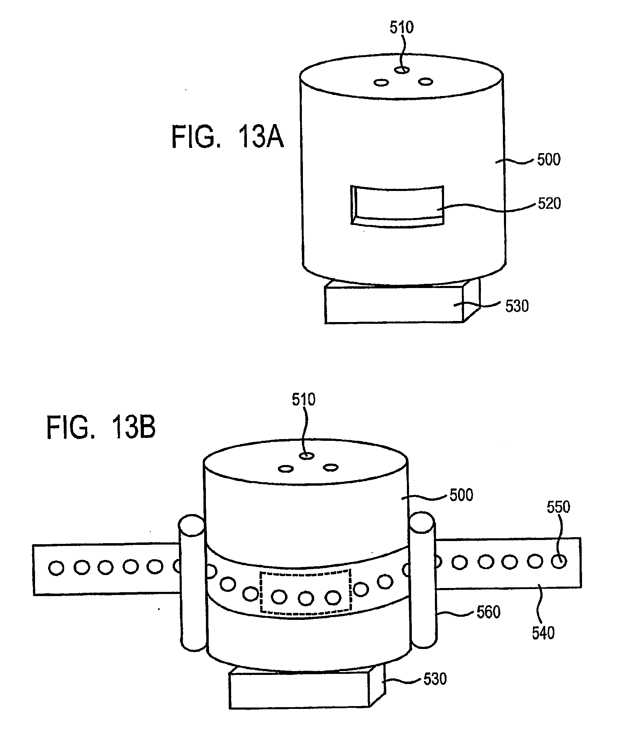

In another embodiment of the invention (illustrated in Fig. 13), multiple blisters or foil pouches containing a drug can be activated by a single vibrator mechanism or piezoelectric element simultaneously by being opened or pierced and exposed to a resonant cavity at the same time prior to the administration of the drug, thus enabling the delivery of a variable dose of the drug by ejecting the drug from the resonant cavity, for example by synthetic jetting in accordance with the teachings of US 2005/0183724-A1.

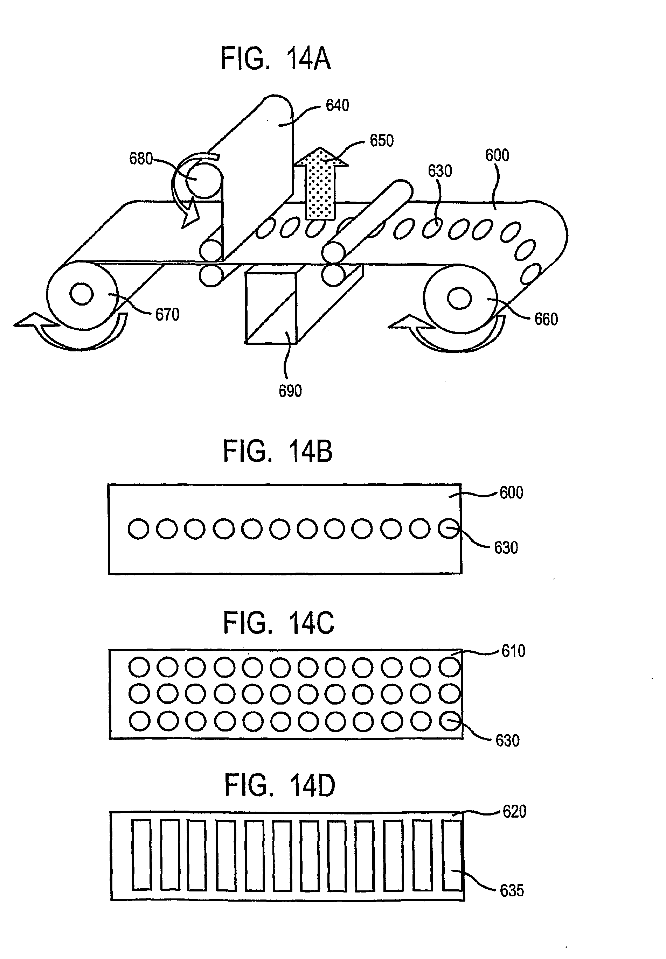

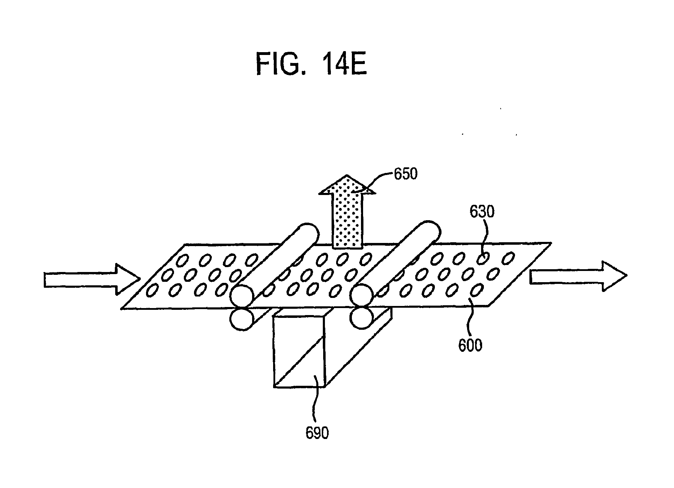

In yet another embodiment of the invention (illustrated in Fig. 14), a variable dose of a drug is delivered to a patient by using at least one vibrator mechanism or piezoelectric element, which is used to simultaneously or sequentially activate multiple selected dose packs so as to result in the delivery of a specific dose of the drug in one inhalation, wherein the dose can be varied according to the patient's needs. In this embodiment a combination of several smaller dose packs results in the controlled total dose meeting a patient's requirements. The dose packs preferably are blisters or foil pouches. According to this embodiment of the invention, the dose packs comprise multiple small cavities or micro-blisters on a foil or within a blister pack which is continuously or intermittently moved during the single inhalation/administration of the drug, passing over the vibrator or piezoelectric element or other mechanical actuator, wherein the variable dose delivered to the patient in one inhalation is defined by the number of the small cavities or micro-blisters which are opened or pierced and subject to administration to the patient during the inhalation. In one embodiment, each micro-blister may contain the same amount of drug, for example, 0.5 mg of the drug. For delivery to the patient of 1 mg of the drug, 2 micro-blisters are opened or pierced. Similarly, for delivery of 2 mg of the drug, 4 micro-blisters are opened or pierced.

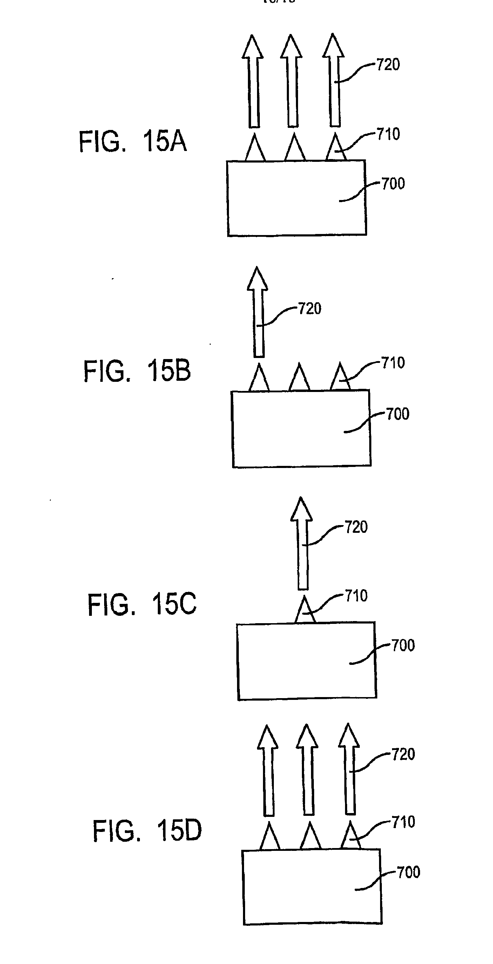

In yet another embodiment of the invention (illustrated in Fig. 15), a variable dose of a drug is delivered to a patient by using at least one vibrator mechanism or piezoelectric element, which is used to simultaneously actuate one or more dose packs. The number of actuated dose packs will determine the total dose delivered to the patient.

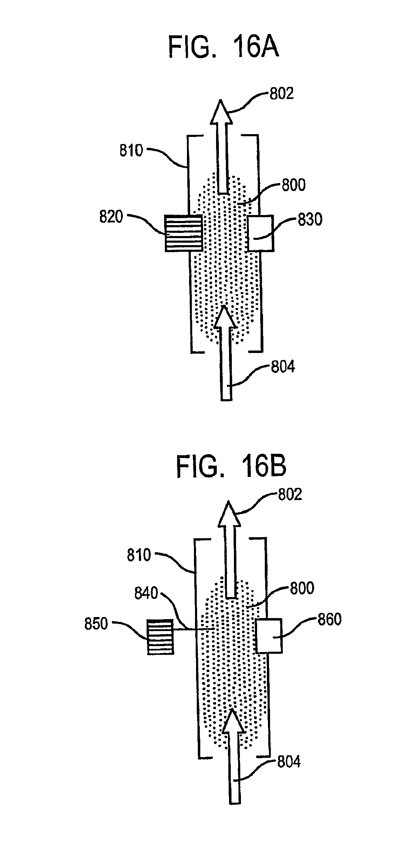

In yet another embodiment of the invention (illustrated in Fig. 16), a sensor is provided for monitoring of the quantity of delivered drug as it is being administered from a dose pack or packs which contain a quantity of the drug exceeding the quantity that the patient needs. The sensor then stops the delivery of the drug once the necessary dose is delivered to the patient and the remaining drug is discarded or retained for future administration. The sensor is preferably an optical or an acoustic sensor capable of detecting and quantifying aerosol particles moving through the flow channel of the inhalation device. In another embodiment the sensor is a sensor which detects the quantity of the drug left in the blister or dose pack or packs, wherein the sensor is preferably a quartz microbalance sensor or piezo sensor or an acoustic sensor. In one embodiment, the piezoelectric element which is used to actuate and vibrate the drug is also utilized as the sensor to detect the quantity of the drug which is left in the blister or dose pack by measuring the resonant frequency of the dose pack or blister pack or electro-mechanical parameters of the piezo actuator, such as admittance of the piezo actuator. In another embodiment, an acoustic sensor is used to detect acoustic properties of the blister or measure the resonant sonic waves generated in the blister and thus monitor the quantity of the drug still remaining in the blister. Once the sensor has detected that the needed quantity of the drug was delivered to the patient, by measuring the remaining quantity of the drug or quantifying the aerosol particles moving through the flow channel, the sensor sends a signal to the controlling circuit to stop the drug delivery to the patient. In another embodiment, the sensor optically detects the quantity of the drug remaining in the dose pack or blister via measurement of optical transmission through the dose pack or blister.

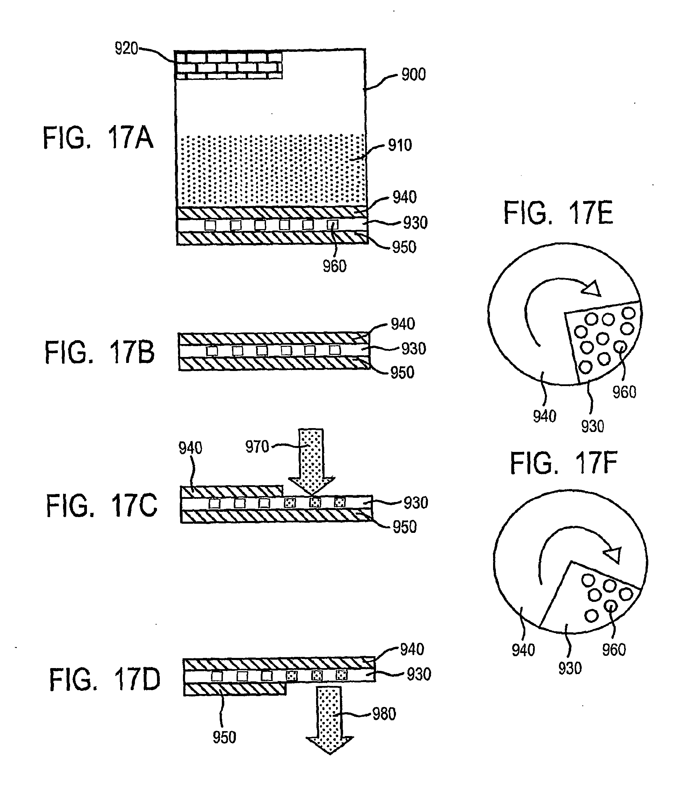

In yet another embodiment of the invention (illustrated in Fig. 17), a canister contains drug quantities sufficient for more than one dosing of the drug. The canister has an outlet communicating with a dosing plate which in a preferred form comprises rotatable disk having micro-dosing cavities of the same or variable size and a first valve plate which in a preferred form comprises a first rotatable lid is located between the canister and the dosing plate to permit selection of the number of cavities for filling with drug, thus permitting selecting a variable dose of the drug. In such embodiment the first valve plate permits opening to a selected number of cavities for filling with the drug from the canister. A second valve plate which in a preferred form comprises a second rotatable disk, is located between the dosing plate and the resonant cavity of an inhaler from which the drug delivery is performed using a vibrator mechanism or piezoelectric element to aerosolize and deliver the drug. In use the first valve plate is opened so as to select a specified number of micro-cavities corresponding to the desired dose. The selected cavities are then filled from the canister. The first valve plate is then closed and the second valve plate is opened permitting the drug to be transferred to the resonant cavity for aerosolization and delivering to the patient by ejection of the drug from the resonant cavity, for example by synthetic jetting in accordance with the teachings of US 2005/0183724-A1.

In still another embodiment of the invention, the delivered dose is estimated from the delivery time and an appropriate calibration curve, wherein the time of the vibrating or piezo actuating of the drug pack or blister is correlated to the delivered dose. In this later embodiment, the necessary dose is delivered by controlling the time of the delivery of the drug or more specifically by controlling the time or duty cycle of activating the vibrator mechanism or the piezo element in contact with the drug pack. In this embodiment either all quantity of the drug contained in an individual drug pack or blister is delivered, for a maximum dose, or partial quantity of the drug contained in an individual drug pack or blister is delivered, for a lower dose of the drug. By switching off the vibrating element before the whole dose contained in an individual drug pack is delivered, a variable dose of the drug can be delivered to a patient. Alternatively, a variable dose of the drug can be delivered to a patient by operating the vibrating element with a lower energy input, resulting in lower vibratory actuation, or operating the vibratory element with a lower duty cycle, intermittently switching the vibratory output on and off.

Other methods, devices, features and advantages of the present invention will be seen from the following drawings and detailed description. It is intended that all such additional methods, devices, features and advantages be included within this description, be within the scope of the present invention, and be protected by the accompanying claims.

Many aspects of the invention can be better understood with reference to the following drawings. The components in the drawings are not necessarily to scale, emphasis instead being placed upon clearly illustrating the principles of the present invention. In the drawings, like reference numerals designate corresponding parts throughout the several views, wherein:
FIG. 1 is a longitudinal cross-sectional schematic view of a first embodiment of inhaler made in accordance with the present invention;
FIG. 2 is a perspective view of the inhaler of Fig. 1;
FIG. 3 is a top perspective view of a pharmaceutical or drug blister pack or cartridge used in the first embodiment;
FIG. 4 is a view similar to FIG. 1 of a second embodiment of the invention;
FIG. 5 is a top plan perspective view of the second embodiment of the invention;
FIG. 6 is a longitudinal cross-sectional schematic view of the second embodiment of the invention;
FIG. 7 is a top perspective view of the third embodiment of the invention;
FIG. 8 is a top perspective view of the cartridge strips used in the third embodiment of the invention of FIG. 7;
FIG. 9 is a top perspective view of the fourth embodiment of the invention;
FIG. 10 is another top perspective view of the fourth embodiment of the invention of FIG. 9;
FIG. 11 is a top perspective view of the pitcher and secondary storage device used in the fourth embodiment of the invention of FIG. 9-10;
FIG. 12 is a top perspective view of the fifth embodiment of the invention and the spool used with the inhaler; and
FIGs. 13-17 illustrate alternative embodiments of the invention.

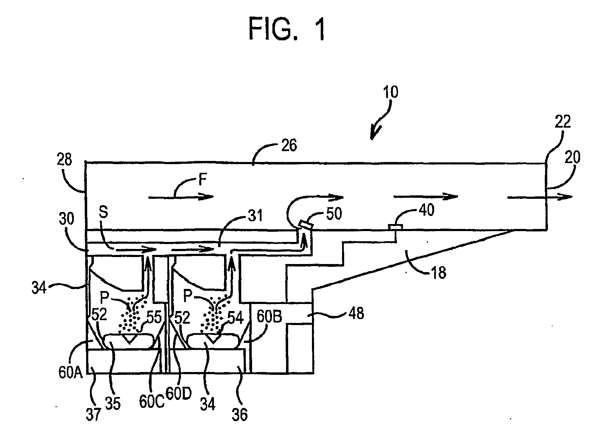

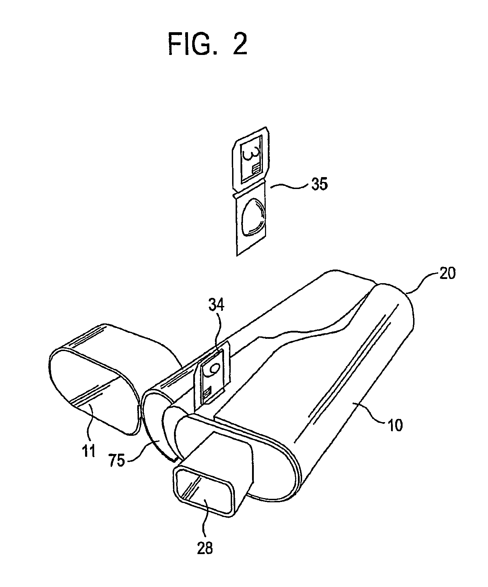

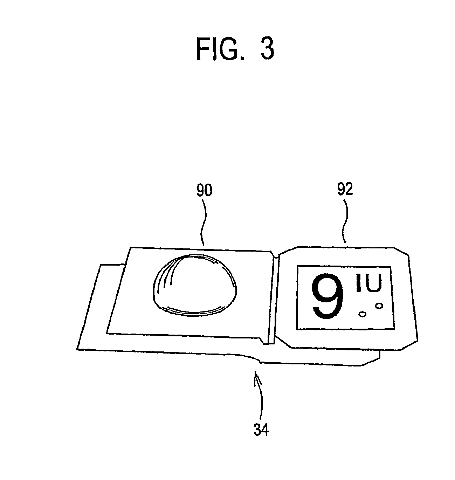

Figures 1-3 illustrate a first embodiment of the present invention. An inhaler 10 includes a hard plastic or metal housing 18 having a generally L-shaped longitudinal cross-section with a mouthpiece cover 11. Housing 18 includes four air flow openings 20, 28, 30, and 32. Inhaler 10 includes a main air flow passage 26 which extends the length of the housing 18 from the front 22 (at opening 20) to the rear 24 thereof (at opening 28) and has a generally square-shaped transverse cross-section, so as to permit air flow through (denoted by arrow F in Figure 3) .

Optional secondary air conduit 31 is generally L-shaped and runs longitudinally from opening 30 in the rear 24 surface of the housing 18 to main passage 26. One-way flow valve 50 is mounted to the inner surface of the main passage 26 via a spring-biased hinge mechanism (not shown), which is adapted to cause the valve 50 to completely block air flow S through the conduit 31 to the main passage 26 when the pressure of the air flow F in the main passage 26 is below a predetermined threshold indicative of inhalation through the passage 26 by a user.

Two powder dispensing chambers 54, 55 are formed in housing 18 for holding a cartridges 34, 35 of powder medication to be inhaled. Housing 18 includes a hingedly moveable panel portion 75 in the rear 24 for permitting the blister packs or cartridges 34, 35 containing a pharmaceutical or drug to be introduced into the two chambers 54, 55 and placed on the seatings 52 of vibration mechanisms 36, 37 between respectively the four guiding means 60A, 60B, 60C, 60D so that cartridges 34, 35 are mechanically coupled to the cartridges 34, 35 to permit maximum vibratory energy to be transmitted from the vibration mechanisms 36, 37 to cartridges 34, 35. Guiding means 60A, 60B, 60C, 60D are designed to allow easy insertion of the cartridges 34, 35 by hand from any secondary packaging (not shown) and retention of the capsule on the seatings 52 in the two chambers 54, 55. Preferably mouthpiece cover 11 is hingedly rotatably attached to panel 75.

Inhaler 10 also preferably includes a conventional miniature air stream velocity or pressure sensor 40 mounted on the inner surface of the conduit 26 so as to sense the speed and/or pressure of the air stream F. Preferably, sensor 40 comprises a springloaded flapper-yield switch which generates electronic signals indicative of the speed and/or pressure of the air stream F in the conduit 26, and transmits those signals for controlling actuation of the vibrator mechanism based upon those signals. Alternatively, sensor 40 may comprise a pressure sensor or an acoustic sensor and control such as described in U.S. Patent No. 6,152,130 assigned to Microdose Technologies, Inc.

Preferably, the control circuitry 48 is embodied as an application specific integrated circuit chip and/or some other type of very highly integrated circuit chip. Alternatively, control circuitry 48 may take the form of a microprocessor, or discrete electrical and electronic components.

The vibration mechanisms 36, 37 preferably are piezoelectric elements, formed of a material that has a high-frequency, preferably, ultrasonic resonant vibratory frequency (e.g., about 10kHz to 100MHz), and are caused to vibrate with a particular frequency and amplitude depending upon the frequency and/or amplitude of excitation electricity applied to the piezoelectric elements 36, 37. Examples of materials that can be used to comprise the piezoelectric elements 36, 37 include quartz and polycrystalline ceramic materials (e.g., barium titanate and lead zirconate titanate). Advantageously, by vibrating the piezoelectric elements 36, 37 at ultrasonic frequencies, the noise associated with vibrating the piezoelectric elements 36, 37 at lower (i.e., non-ultrasonic) frequencies can be avoided.

An embodiment of the inhaler without optional air conduit 30 and without air flow opening 30 and valve 50 is also disclosed in the present invention. In this embodiment drug powder is discharged directly into the main air flow channel 26.

In this first embodiment of the present invention, the drug is stored as unit doses in individual blister packs 34, 35. Referring in particular to FIG. 3, the individual blister packs 34, 35 contain two parts: a blister 90 and a labeled substrate 92. The blister 90 contains controlled aliquots or doses of a dry powder medication or a liquid drug. The labeled substrate 92 serves several purposes: it provides information about what type and the amount of drug or medication in the blister; it supports the blister; and it provides a handle for easy loading of the blister packs 34, 35 into the inhaler 10. A large number or other indicia (in this case, the number "9^{iu}") on the label 92 indicates the dose size contained in the blister pack. For example, the number "9^{iu}" indicates the blister pack contains 9 units of insulin. Other size dose packs, e.g., a 3 unit pack would permit the user to select a dose of 3, 6, 9 or 12 units in a single puff by selecting one or combining two blister packs. Similarly, blister packs containing 1, 2 and 4 units would permit the user to select a dose of 1, 2, 3, 4, 5, 6 and 8 units in a single puff by selecting one or combining two blister packs. In like manner, blister packs containing 3, 4 and 5 units would permit the user to select a dose of 3, 4, 5, 6, 7, 8, 9 or 10 units in a single puff by selecting one or combining two blister packs. The large numbering allows the user to easily calculate the desired combination of blister packs to insert into the inhaler. The blister packs 34, 35 also may contain an electronically or mechanically readable label or tag; the label or tag containing information about the contents of the blister. The inhaler may include a mechanism to read this information to check that the user receives the correct dose of the correct drug.

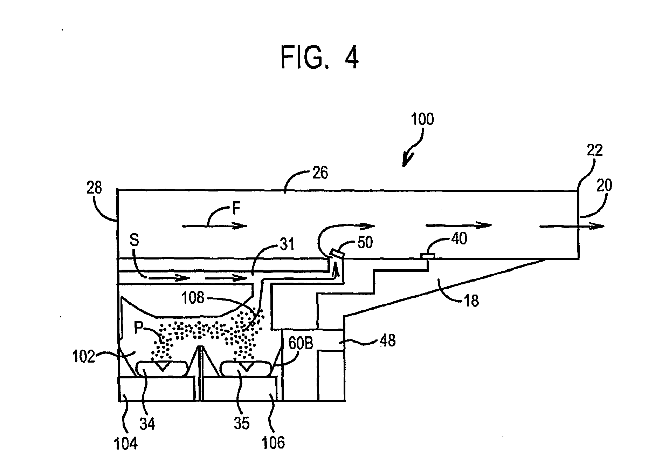

A second preferred embodiment 100 of the present invention is shown in FIG. 4. In this embodiment, the inhaler 100 only contains one powder dispensing chamber 102. Chamber 102 contains two vibration mechanisms 104, 106, which allow two blister packs 34, 35 to be placed on the seating of vibration mechanisms 104, 106. The air flow P including the drug from both cartridges 34, 35 flow through passageway 108 through the conduit 31 to the main passage 26.

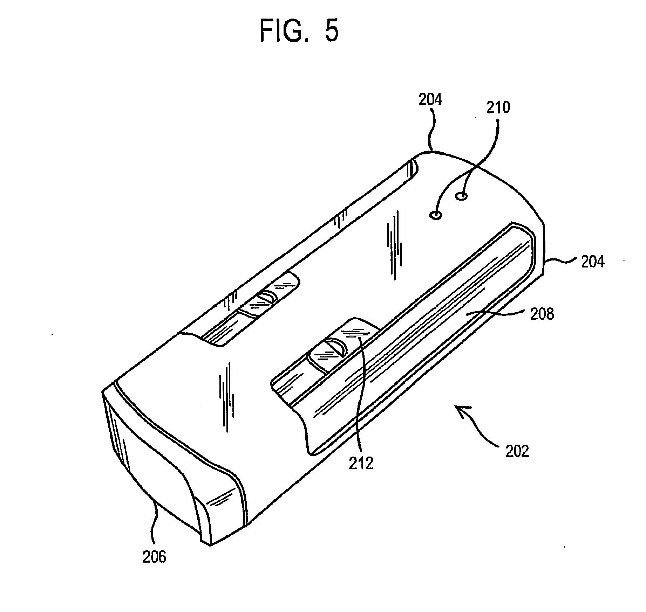

FIG. 5-6 illustrate a third embodiment 202 of the present invention. In this embodiment, the inhaler is designed to accommodate a pair of cartridge strips only one of which 214 is shown, that are inserted into a slot (not shown) in the back 204 of the inhaler 202. A mouthpiece cover 206 (shown covered) is hingedly rotatably attached over a mouthpiece (not shown) at the front of the inhaler. Each cartridge strip carries a plurality of blister packs 34. Preferably, all of the blister packs 34 on a particular strip contain similar amounts of medication. The user controls the desired dosage of the medicine or drug by loading two cartridge strips having different blister pack loadings into the inhaler, and sliding the buttons 212 on the top of the inhaler 202 to access and pierce one or several blister packs on each strip. Once the user has selected the desired dosage and the blisters 90 have been pierced, and the piezoelectric vibrators deaggregate the medication. Preferably, a flow sensor and feedback such as a noise generator or one or more lights 210 may be provided, e.g., as described in published U.S. application no. US 2003/0041859-A1, to inform the user when the medicine is inhaled correctly and when the dosing is complete. Depending on the total dosage required, the user might need to switch cartridge strips and inhale again or take additional inhalations with the same cartridge. After the user has inhaled, the respective strips are advanced, e.g. like a film camera, past the used blisters. Preferably, the covering 208 around strips 214 is made transparent so as to allow the user to observe when the strips 214 are empty.

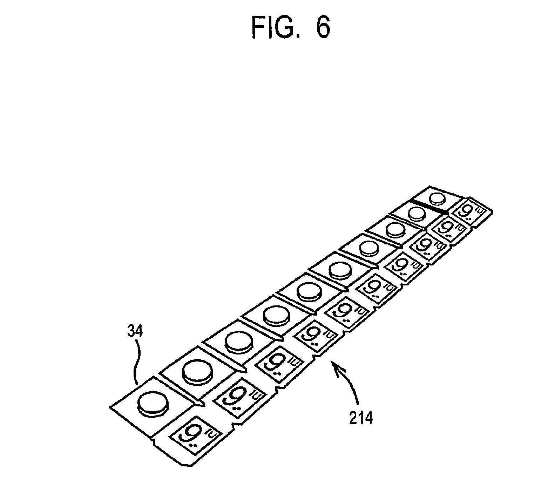

FIG. 6 shows a cartridge strip 214 consistent with the third preferred embodiment of the present invention. The cartridge strip 214 consists of multiple cartridges 34 with the labels or indicia printed thereon.

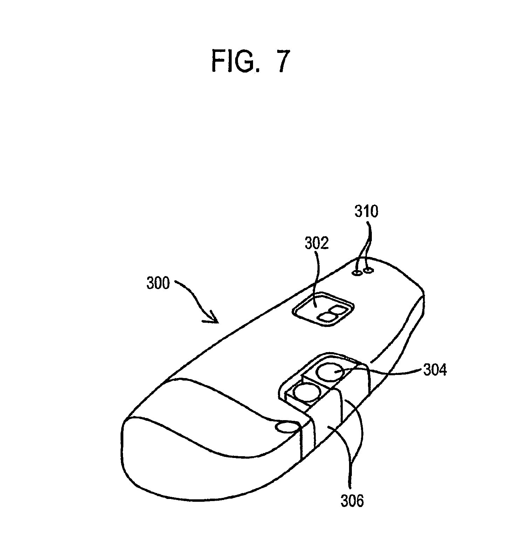

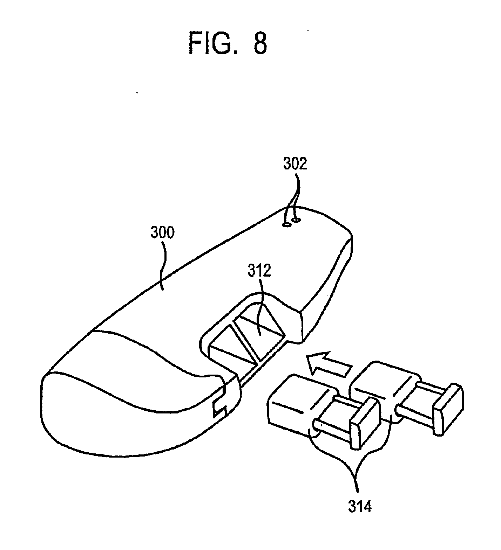

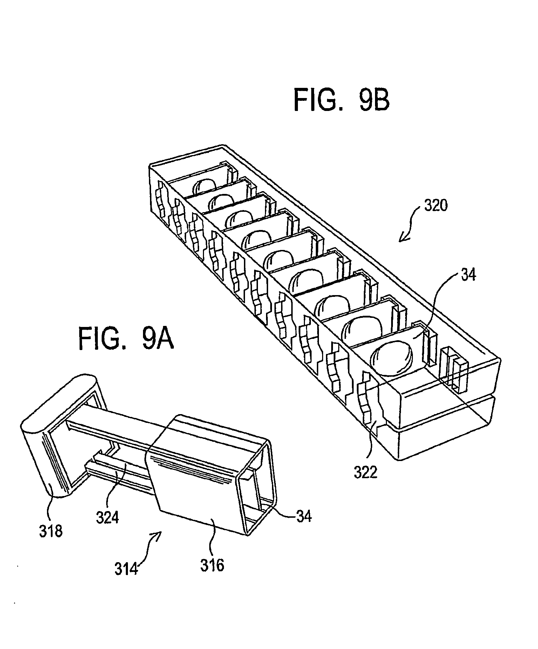

A fourth embodiment 300 of the present invention, as shown in FIG. 7-9, permits the user to select individual blisters 90 or combinations thereof from a protective cartridge, and to insert the one or two blisters 90 depending on the dose of drug required into receiving slots 312 in an inhaler 300 using a fixture or tool 314. As described earlier, the inhaler may include a flow sensor and feedback such as a noise generator or lights 310 to inform the user when the medicine is inhaled correctly and when the dosing in complete. Also, if the user is not inhaling correctly, the inhaler 300 can be programmed to stop dosing until the user is inhaling correctly. The inhaler 300 also may be programmed to sum the number of blisters dispensed and keep a running total for the duration of the dosing event, and to display the total on an LCD 302 or the like.

In this embodiment, a secondary packaging device or protective cartridge 320 protects and stores the individual blisters 90 before use. The secondary packaging device 320 contains slots 322 to hold the blisters 90. Movement of the blisters 90 from the secondary packaging device 320 to the inhaler 300 is accomplished by using a fixture or tool 314. Fixture or tool 312 preferably includes a pair of parallel tracks 324 with a groove to allow easy capture of blisters 90. A protective shield 316 on the fixture or tool 314 protects the blister 90 as it is transported between the cartridge and the tool in use. The fixture or tool 314 is inserted into the cartridge 320 through slot 322 to grab a blister 34. The user then withdraws the fixture or tool 314 and moves it to and inserts it into an opening 312 of the inhaler 300. The fixture or tool 314 is left in place while the inhaler is used. The fixture or tool 314 is then removed, taking the spent blister with it. A feature and advantage of using the fixture or tool is that contamination or possible damage to the blister caused by contact with the user's hand or fingers may be avoided.

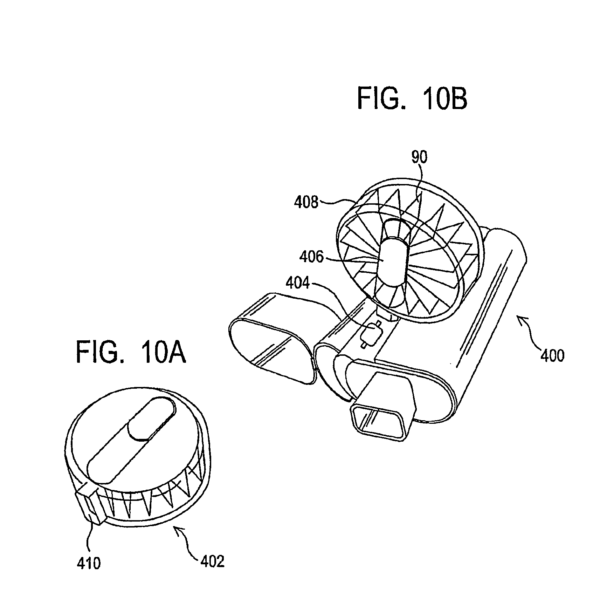

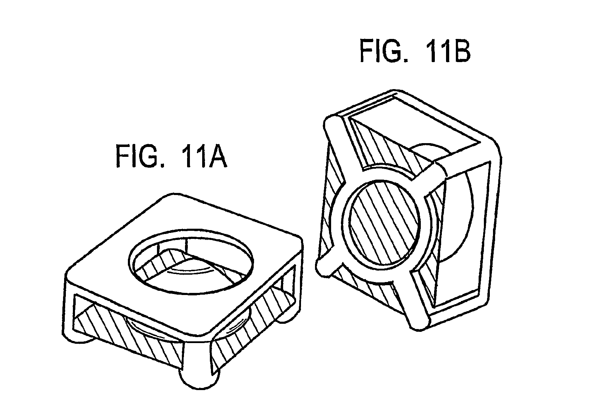

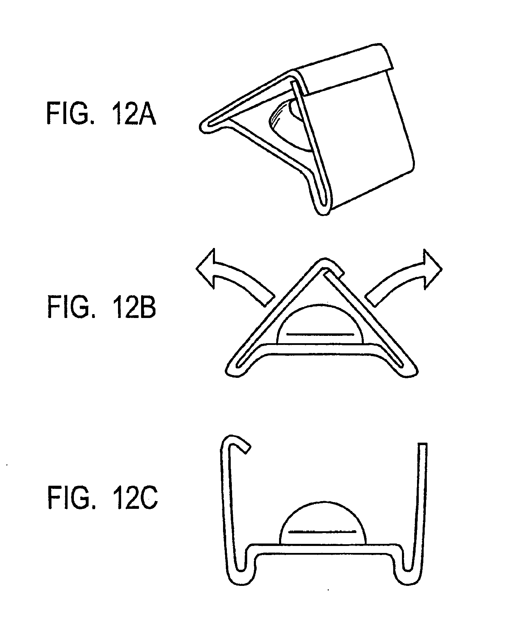

A fifth embodiment of the present invention uses a spool or carousel 402 to protect blisters 90 before delivery, as illustrated in FIG. 10. In use, carousel 402 is mounted to a slot 404 in the inhaler 400. The carousel 402 is rotated to deliver a blister 90 to opening 410. The blisters 90 then can drop from the slot 404 through the opening 410 into the inhaler where they can be opened and processed as before. The blisters contained in the spool carousel each contains the same dosage of a drug. Other packaging techniques and structures for protecting blisters are illustrated in FIGs. 11-12.

Referring now to Figs. 13A and 13B, an embodiment of the present invention includes resonant cavity 500 capable of aerosolizing and ejecting the drug substance from drug ejection apertures 510, upon actuation by the vibrator 530, such a piezo actuator or transducer, which is coupled to resonant cavity 500. A dose pack or blister delivery window 520 is provided for depositing variable quantity of drug substance into the resonant cavity 500. Blister tape 540 is engaged by tape advancement mechanism 560 and is advanced prior to dosing to bring drug-containing dose packs or blisters in contact with the delivery window 520. In this embodiment, a selected number of blisters 550 on a blister tape 540 are pierced or opened to result delivery of a desired dose of the drug. In this embodiment, multiple dose packs 550 are activated by one vibrator 530 simultaneously by being opened and exposed to resonant cavity 520 at the same time prior to the administration of the drug, thus enabling the delivery of a variable dose of the drug by ejecting the drug from the resonant cavity, for example by synthetic jetting.

Referring now to Fig 14A, in another embodiment of the invention a variable dose of a drug is delivered to a patient by using at least one vibrator 690, such as piezoelectric element, which simultaneously or sequentially activates multiple selected dose packs 630 or 635 so as to result in the delivery of a specific desired dose of the drug, preferably in one inhalation. The delivered dose can be varied according to the patient's needs by selecting one or more of dose packs 630 or 635. Dose packs 630 and 635 are arranged on a tape 600, 610, or 620 in one or several rows as illustrated in Figs. 14B and 14C, and can be of variable shapes, such as round dose packs 630 or elongated dose packs 635 as illustrated in Fig. 14D. Dose packs 630 and 635 are preferably blisters or similar compartments formed in the carrier tape 600, 610, or 620 capable of holding a predetermined amount of drug. In one embodiment, tape 600 is being moved across the surface of the vibrator 690 continuously or intermittently with the lidding tape 680 peeled from the individual dose packs 630 by the peeling mechanism 680. Tape 600 is advanced by tape advancement mechanism 660 from spool 670. Arrow 650 indicates the direction of movement of the ejected and aerosolized drug upon actuation of vibrator 690. The dose of the drug delivered to the patient is controlled by the number of dose packs 630 opened and in contact with the piezo actuator during the drug delivery event. According to this embodiment of the invention, dose packs 630 or 635 comprise multiple small cavities or micro-blisters on a tape or foil or within a blister pack which is continuously or intermittently moved during the single inhalation/administration of the drug, passing over the vibrator or piezoelectric element or other mechanical actuator, wherein the variable dose delivered to the patient in one inhalation is defined by the number of the small cavities or micro-blisters which are opened or pierced and subject to administration to the patient during the inhalation. In one embodiment, each micro-blister or dose pack 630 may contain the same amount of drug, for example, 0.5 mg of the drug. For delivery to the patient of 1 mg of the drug, 2 micro-blisters are opened or pierced. Similarly, for delivery of 2 mg of the drug, 4 micro-blisters are opened or pierced.

Referring now to Fig. 14E, an embodiment of the present invention is shown wherein a selected number of dose packs or micro-blisters 630 are opened by piercing of the top cover of dose pack, thus enabling ejection of the drug upon contact with vibrator 690 (piercing mechanism not shown). In this embodiment, a plurality of micro-blisters or dose packs 690 are in contact with vibrator 690 during the dosing event. The ejection of the drug proceeds only from pierced or opened micro-blisters or dose packs 690, thus selection of the number of pierced or opened micro-blisters or dose packs 690 defines the variable dose of the drug to be delivered to a patient. Arrow 650 indicates the direction of movement of the ejected and aerosolized drug upon actuation of vibrator 690.

Referring now to Figs. 15A and 15B, in another embodiment of the present invention, a variable dose of a drug is delivered to a patient by using at least one vibrator 700, which is used to simultaneously actuate one or more dose packs 710. The number of actuated dose packs will determine the total dose delivered to the patient. Fig. 15A illustrates delivery of a large quantity of drug from a plurality of pierced or opened dose packs 710, with aerosolization and ejection of the drug schematically shown by arrows 720.
Fig. 15B illustrates delivery of a small quantity of drug from one pierced or opened dose pack 710, with aerosolization and ejection of the drug is schematically shown by arrow 720. In this embodiment, variable dose of the drug is defined by the number of dose packs or blisters 710 which are pierced or opened.

In another embodiment of the present invention shown in Fig. 15C and 15D, delivery of variable dose of the drug is performed by selecting the number of individual dose packs or blisters 710 which are pierced or opened and are all coupled to vibrator 700. Fig. 15C illustrates one individual dose pack 710 and Fig. 15D illustrates three individual dose packs 710, with aerosolization and ejection of the drug schematically shown by arrows 720.

Figs. 16A and 16B illustrate another embodiment of the present invention in which a sensor or detector is provided for monitoring of the quantity of delivered drug. The drug is being ejected from a dose pack or packs which contain a quantity of the drug exceeding the quantity that the patient needs. The delivery of the drug is stopped once the necessary dose is delivered to the patient and the remaining drug is discarded or retained for future administration, resulting in delivery of a variable dose of the drug. The delivery of the drug is stopped by discontinuing actuation of the vibrator, such as piezo vibrator, providing the vibratory energy to the dose pack or blister. The sensor is preferably an optical or an acoustic sensor capable of detecting and quantifying aerosol particles moving through the flow channel of the inhalation device.

As illustrated in Figs. 16A and 16B, plume of aerosolized drug 800, which can also be a drug mixed with excipients, is moving through the inhaler flow channel 810 as shown by arrows 804 and 802. Referring now to Fig. 16A, aerosol 800 passes by an optical, acoustic, or other physical sensor or detector capable of measuring the properties of aerosol plume 800 and inferring the quantity of the drug which has passed through the flow channel 810. Optical or acoustic source 820 is shown installed in flow channel 810, whereas optical or acoustic detector 830, also installed in flow channel 810, is capable of detecting the attenuation of the signal emitted by source 820 due to interaction with aerosol 800. The attenuation of the signal, integrated over the time of aerosol passing through flow channel 810, enables to infer the quantity of the drug which has passed through the flow channel 810. After a predetermined dose has passed thorough flow channel 810, actuation of the piezo actuator (not shown) is stopped and thus drug delivery is discontinued. Thus a variable dose of the drug can be delivered. In another embodiment, instead of optical or acoustic detector 830, a reflector is installed (not shown), capable of reflecting attenuated optical or acoustic signal back to optical or acoustic source 820, which is in this embodiment is also capable of receiving the reflected signal, as known in the art. The attenuation of the signal, integrated over the time of aerosol passing through flow channel 810, enables to infer the quantity of the drug which has passed through the flow channel 810.

Referring now to Fig. 16B, there is provided an optical source 850 installed outside of flow channel 810, with a fiberoptic guide or optical fiber or optical conduit 840 entering flow channel 810. Optical signal exiting optical fiber 840 is attenuated by aerosol 800 and is detected by optical detector 860. The signal, integrated over the time of aerosol passing through flow channel 810, enables to infer the quantity of the drug which has passed through the flow channel 810. After a necessary dose of the drug has passed thorough flow channel 810, actuation of the piezo actuator (not shown) is stopped and thus drug delivery is discontinued. Thus a variable dose of the drug can be delivered.

Alternatively, the sensor is a sensor which detects the quantity of the drug left in the blister or dose pack or packs, wherein the sensor is preferably a quartz microbalance sensor or a piezo sensor or an acoustic sensor. In one embodiment, the piezoelectric element which is used to actuate and vibrate the blister for ejection of the drug is also utilized as the sensor to detect the quantity of the drug which is left in the blister or dose pack by measuring the resonant frequency or electro-mechanical parameters of the piezo actuator, such as admittance of the piezo actuator. In yet another embodiment, an acoustic sensor is used to detect acoustic properties of the blister or measure the resonant sonic waves generated in the blister and thus monitor the quantity of the drug still remaining in the blister. In still yet another embodiment, the sensor optically detects the quantity of the drug remaining in the dose pack or blister via measurement of optical transmission through the dose pack or blister. Once the sensor has detected that the needed quantity of the drug was delivered to the patient, through measuring the remaining quantity of the drug or quantifying the aerosol particles moving through the flow channel, the sensor sends a signal to the controlling circuit to stop the drug delivery to the patient.

Referring now to Figs. 17A-17F, in another embodiment of the invention, a canister 900 contains bulk, i.e., multi-dose quantities of a drug. An optional hygroscopic element 920 may be included in the canister to absorb moisture and keep optimal level of humidity inside canister 900. Canister 900 has an outlet communicating with a dosing plate 930 which in a preferred form comprises rotatable disk having micro-dosing cavities 960 of the same or variable size and a first valve plate 940 which in a preferred form comprises a first rotatable lid that is located between the canister and the dosing plate to permit selection of the number of cavities for filling with drug, thus permitting selecting a variable dose of the drug. In such embodiment the first valve plate 940 permits opening to a selected number of cavities for filling with the drug from the canister 900. A second valve plate 950 which in a preferred form comprises a second rotatable disk is located between the dosing plate and the resonant cavity of an inhaler from which the drug delivery is performed using a vibrator mechanism or piezoelectric element to aerosolize and deliver the drug. In use the first valve plate 940 is opened so as to select a specified number of micro-cavities 960 corresponding to the desired dose. The selected cavities are then filled from the canister 900 as schematically shown by arrow 970. The first valve plate 940 is then closed and the second valve plate 950 is opened permitting the drug to be transferred as schematically shown by arrow 980 to the resonant cavity (not shown) for aerosolization and delivering to the patient by ejection of the drug from the resonant cavity, for example by synthetic jetting. Figs. 17B through 17C show the dosing plate 930 closed, open for filling with powder 910, and open for discharging powder 910 respectively. Figs 17E and 17F are top plan views of dosing plate 930 shown with first valve plate 940 open for selecting a variable dose of the drug powder 910 through selection of a variable number of micro-dosing cavities 960.

In still another embodiment of the invention, there is provided an inhaler similar to inhaler 10 shown in Fig. 1 or inhaler 100 shown in Fig. 4, but with only one vibration mechanism 36 or 37 or 104 or 106. The delivered dose from a single cartridge 34 or 35 coupled to vibration mechanism is estimated from the delivery time and an appropriate calibration curve, wherein time of the vibrating or piezo actuating of the cartridge 34 or 35 which can be a drug pack or a blister is correlated to the delivered dose. In this later embodiment, the necessary dose is delivered by controlling the time of the delivery of the drug or more specifically by controlling the time or duty cycle of activating the vibrator mechanism or the piezo element in contact with the drug pack. In this embodiment either all quantity of the drug contained in an individual drug pack or blister is delivered, for a maximum dose, or partial quantity of the drug contained in an individual drug pack or blister is delivered, for a lower dose of the drug. By switching off the vibrating element before the whole dose contained in an individual drug pack is delivered, a variable dose of the drug can be delivered to a patient. Alternatively, a variable dose of the drug can be delivered to a patient by operating the vibrating element with a lower energy input, resulting in lower vibratory actuation, or operating the vibratory element with a lower duty cycle, intermittently switching the vibratory output on and off.

The above-described embodiments of the present invention are merely possible examples of implementations, merely set forth for a clear understanding of the principles of the invention. Many variations and modifications may be made to the above-described embodiment(s) of the invention without departing substantially from the spirit and principles of the invention.

## Claims

1. A dry powder inhalation device (10) for delivering a therapeutic agent to a patient wherein the therapeutic agent is a dry powder (P) that is deaggregated by a vibrator and picked up in an air stream (F) for delivery to the patient, wherein the inhalation device (10) comprises at least two vibrators (36, 37) for selectively coupling with two or more dose packs, **characterized in that** the two or more dose packs contain the same or different dose unit quantities of said therapeutic agent which dose packs are combined to make up a desired dose of said therapeutic agent.

2. The inhalation device according to claim 1, **characterized in that** said at least two vibrators (36, 37) vibrate at the same frequency and/or amplitude.

3. The inhalation device according to claim 1, **characterized in that** said at least two vibrators (36, 37) vibrate at different frequencies and/or amplitudes.

4. The inhalation device according to claim 1, **characterized in that** said at least two vibrators (36, 37) comprise piezoelectric vibrators.

5. The inhalation device according to claim 1, **characterized in that** said at least two vibrators (36, 37) comprise electrostatically driven diaphragms.

6. The inhalation device according to claim 1, **characterized in that** said dose packs comprise blister packs (34, 35) containing measured quantities of said therapeutic agent.

7. The inhalation device according to claim 6, **characterized in that** a plurality of said blister packs (34, 35) are carried on a strip.

8. The inhalation device according to claim 1, **characterized in that** the dose packs comprise dose sizes selected from the group consisting of 1, 2, 3, 4 and 5 dose units.

9. The inhalation device according to claim 8, **characterized in that** the therapeutic agent comprises insulin, and the dose packs comprise 1 unit dose pack of insulin, 2 unit dose packs of insulin, 3 unit dose packs of insulin, 4 unit dose packs of insulin, and 5 unit dose packs of insulin.

## Patentansprüche

1. Eine Inhalationsvorrichtung (10) für ein trockenes Pulver zum Verabreichen eines therapeutischen Wirkstoffs an einen Patienten, wobei der therapeutische Wirkstoff ein trockenes Pulver (P) ist, das durch einen Vibrator verteilt wird und in einem Luftstrom (F) zur Verabreichung an den Patienten aufgelesen wird, wobei die Inhalationsvorrichtung (10) zumindest zwei Vibratoren (36, 37) zum selektiven Zusammenwirken mit zwei oder mehr Dosispackungen aufweist,
**dadurch gekennzeichnet, dass**
die zwei oder mehr Dosispackungen dieselben oder verschiedene Dosiseinheitsmengen des besagten therapeutischen Wirkstoffs enthalten, wobei die Dosispackungen kombiniert werden, um eine gewünschte Dosis des besagten therapeutischen Wirkstoffs zu ergeben.

2. Die Inhalationsvorrichtung nach Anspruch 1,
**dadurch gekennzeichnet, dass**
die besagten mindestens zwei Vibratoren (36, 37) bei derselben Frequenz und/oder derselben Amplitude vibrieren.

3. Die Inhalationsvorrichtung nach Anspruch 1,
**dadurch gekennzeichnet, dass**
die besagten mindestens zwei Vibratoren (36, 37) bei verschiedenen Frequenzen und/oder verschiedenen Amplituden vibrieren.

4. Die Inhalationsvorrichtung nach Anspruch 1,
**dadurch gekennzeichnet, dass**
die besagten mindestens zwei Vibratoren (36, 37) piezoelektrische Vibratoren umfassen.

5. Die Inhalationsvorrichtung nach Anspruch 1,
**dadurch gekennzeichnet, dass**
die besagten mindestens zwei Vibratoren (36, 37) elektrostatisch betriebene Diaphragmen umfassen.

6. Die Inhalationsvorrichtung nach Anspruch 1,
**dadurch gekennzeichnet, dass**
die besagten Dosispackungen Blisterpackungen (34, 35) umfassen, die gemessene Mengen des besagten therapeutischen Wirkstoffs enthalten.

7. Die Inhalationsvorrichtung nach Anspruch 6,
**dadurch gekennzeichnet, dass**
eine Vielzahl der besagten Blisterpackungen (34, 35) auf einem Streifen getragen wird.

8. Die Inhalationsvorrichtung nach Anspruch 1,
**dadurch gekennzeichnet, dass**
die Dosispackungen Dosisgrößen umfassen, die aus der Gruppe bestehend aus ein, zwei, drei, vier und fünf Dosiseinheiten ausgewählt sind.

9. Die Inhalationsvorrichtung nach Anspruch 8,
**dadurch gekennzeichnet, dass**
der therapeutische Wirkstoff Insulin umfasst und dass die Dosispackungen eine Einheitsdosispackung Insulin, zwei Einheitsdosispackungen Insulin, drei Einheitsdosispackungen Insulin, vier Einheitsdosispackungen Insulin und fünf Einheitsdosispackungen Insulin umfassen.

## Revendications

1. Dispositif d'inhalation de poudre sèche (10) pour la délivrance d'un agent thérapeutique à un patient, sachant que l'agent thérapeutique est une poudre sèche (P) qui est désagrégée par un vibrateur et entraînée dans un courant d'air (F) pour la délivrance au patient, que le dispositif d'inhalation (10) comporte au moins deux vibrateurs (36, 37) pour se coupler sélectivement à deux ou plus de deux emballages de dose, **caractérisé en ce que** les deux ou plus de deux emballages de dose contiennent la même quantité de dose unitaire ou des quantités de dose unitaire différentes dudit agent thérapeutique, lesquels emballages de dose sont combinés pour constituer une dose souhaitée dudit agent thérapeutique.

2. Dispositif d'inhalation selon la revendication 1, **caractérisé en ce que** les au moins deux vibrateurs (36, 37) vibrent à la même fréquence et/ou amplitude.

3. Dispositif d'inhalation selon la revendication 1, **caractérisé en ce que** les au moins deux vibrateurs (36, 37) vibrent à des fréquences et/ou amplitudes différentes.

4. Dispositif d'inhalation selon la revendication 1, **caractérisé en ce que** les au moins deux vibrateurs (36, 37) comportent des vibrateurs piézoélectriques.

5. Dispositif d'inhalation selon la revendication 1, **caractérisé en ce que** les au moins deux vibrateurs (36, 37) comportent des diaphragmes à entraînement électrostatique.

6. Dispositif d'inhalation selon la revendication 1, **caractérisé en ce que** lesdits emballages de dose comportent des emballages alvéolaires (34, 35) contenant des quantités mesurées dudit agent thérapeutique.

7. Dispositif d'inhalation selon la revendication 6, **caractérisé en ce qu'**une multitude desdits emballages alvéolaires (34, 35) est portée sur une plaquette alvéolée.

8. Dispositif d'inhalation selon la revendication 1, **caractérisé en ce que** les emballages de dose présentent des quantités de dose sélectionnées dans le groupe constitué par 1, 2, 3, 4 et 5 doses unitaires.

9. Dispositif d'inhalation selon la revendication 8, **caractérisé en ce que** l'agent thérapeutique comprend de l'insuline et que les emballages de dose comprennent 1 emballage de dose unitaire d'insuline, 2 emballages de dose unitaire d'insuline, 3 emballages de dose unitaire d'insuline, 4 emballages de dose unitaire d'insuline et 5 emballages de dose unitaire d'insuline.
